# EUROPÄISCHE PATENTSCHRIFT

(11) **EP 0 546 420 B1**
(45) Veröffentlichungstag und Bekanntmachung des Hinweises auf die Patenterteilung: **05.06.1996**
(21) Anmeldenummer: 92120437.6
(22) Anmeldetag: 30.11.1992
(51) Int. Cl.: C07D 231/16

(54) **Verfahren zur Herstellung von substituierten Pyrazolinen**
Process for the preparation of substituted Pyrazolines
Procédé pour la préparation de pyrazolines substituées

(30) Priorität: 13.12.1991 DE 4141187
(43) Veröffentlichungstag der Anmeldung: 16.06.1993
(73) Patentinhaber: BAYER AG, 51368 Leverkusen (DE)
(72) Erfinder: Gallenkamp, Bernd, Dr., W-5600 Wuppertal 1 (DE); Fuchs, Rainer, Dr., W-5600 Wuppertal 1 (DE)

(56) Entgegenhaltungen:
- EP-A- 0 438 690
- SYNTHETIC COMMUNICATIONS Bd. 17, Nr. 7, 1987, Seiten 809 - 815 K. TAKAHASHI; M. OGATA 'A convenient synthesis of 1H-1,2,4-Triazol-1-yl propan-3-one derivatives by modified Mannich reaction'
- SYNTHETIC COMMUNICATIONS Bd. 14, Nr. 6, 1987, Seiten 585 - 590 K. MATSUMOTO ET AL. 'Mannich reaction under high pressure. Dimethylaminomethylation of ketones with bis(dimethylamino)methane under mild conditions'

## Beschreibung

Die Erfindung betrifft ein neues Verfahren und neue Zwischenprodukte zur Herstellung von bekannten substituierten Pyrazolinen, welche als Zwischenprodukte für Schädlingsbekämpfungsmittel verwendet werden können.

Es ist bekannt, dar man substituierte Pyrazoline erhält, wenn man substituierte Alkenylketone mit Hydrazin(hydrat) umsetzt (vgl. EP-A 438690). Bei diesem Verfahren werden jedoch die substituierten Pyrazoline - wie auch die als Vorprodukte benötigten Alkenylketone - nur in mäßigen Ausbeuten erhalten. Die Ausbeuten verschlechtern sich zudem mit zunehmender Ansatzgröße, sodaß eine entsprechende großtechnische Darstellung praktisch nicht möglich ist.

Es wurde nun gefunden, daß man die substituierten Pyrazoline der allgemeinen Formel (I) 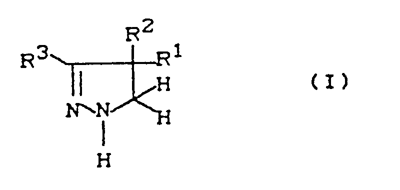
in welcher
- R¹ und R³: gleich oder verschieden sind und jeweils für gegebenenfalls substituiertes Aryl oder für gegebenenfalls substituiertes und/oder benzanelliertes Heterocyclyl mit 5 oder 6 Ringgliedern, von denen 1 bis 4 Stickstoffatome und die restlichen Kohlenstoffatome sind, stehen, und
- R²: für Wasserstoff oder für jeweils gegebenenfalls substituiertes Alkyl, Cycloalkyl oder Aryl steht,
in sehr guten Ausbeuten und in sehr guter Qualität erhalt, wenn man in einer ersten Reaktionsstufe eine Mischung bestehend aus einem substituierten Keton der allgemeinen Formel (II) 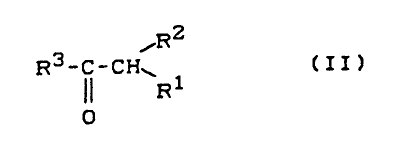
in welcher
- R¹, R² und R³: die oben angegebene Bedeutung haben,
und einem Bis-dialkylaminomethan der allgemeinen Formel (III)

(R⁴)₂N-CH₂-N(R⁴)₂ (III)

in welcher
- R⁴: für Alkyl steht,
gegebenenfalls in Gegenwart eines Verdünnungsmittels bei Temperaturen zwischen 0°C und 100°C umsetzt und die hierbei gebildeten substituierten Dialkylaminoalkylketone der allgemeinen Formel (IV) 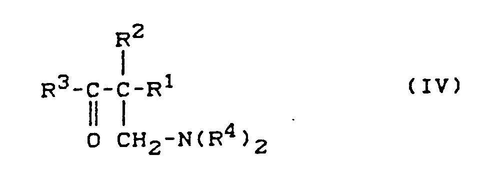
in welcher
- R¹, R², R³ und R⁴: die oben angegebene Bedeutung haben,
gegebenenfalls isoliert und in einer zweiten Verfahrensstufe mit Hydrazin(hydrat) gegebenenfalls in Gegenwart eines Verdünnungsmittels bei Temperaturen zwischen 0°C und 100°C umsetzt.

Überraschenderweise können nach dem erfindungsgemäßen Verfahren die substituierten Pyrazoline der Formel (I) in wesentlich höheren Ausbeuten und in besserer Qualität als nach der bekannten Verfahrensweise gemäß EP-A 438 690 erhalten werden. Im Gegensatz zum bekannten Verfahren ist das erfindungsgemäße Verfahren problemlos den technischen Maßstab übertragbar. Das erfindungsgemäße Verfahren stellt somit eine wertvolle Bereicherung des Standes der Technik dar.

Verwendet man beispielsweise α-(Pyrazol-1-yl)-4-fluoracetophenon und Bis-dimethylamino-methan als Ausgangsstoffe, so kann der Reaktionsablauf beim erfindungsgemäßen Verfahren durch das folgende Formelschema skizziert werden: 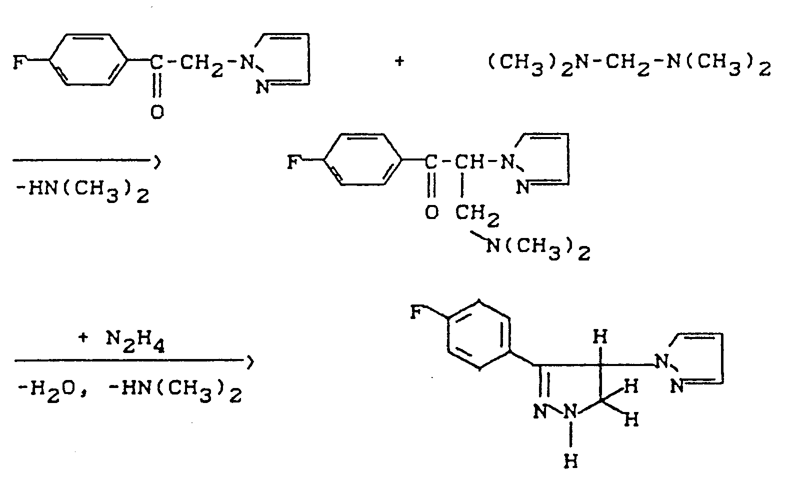
Nach dem erfindungsgemäßen Verfahren werden vorzugsweise Verbindungen der Formel (I) hergestellt, in welcher
- R¹ und R³: gleich oder verschieden sind und jeweils für gegebenenfalls durch Halogen, Cyano, Nitro, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Di-(C₁-C₄-alkyl)-amino, C₁-C₆-Alkoxy-carbonyl, C₁-C₆-Halogenalkoxy-carbonyl, C₁-C₂-Alkylendioxy, C₁-C₂-Halogenalkylendioxy, Phenoxy (welches gegebenenfalls durch Halogen und/oder C₁-C₄-Halogenalkyl substituiert ist) oder Phenylthio (welches gegebenenfalls durch Halogen und/oder C₁-C₄-Halogenalkyl substituiert ist) substituiertes Phenyl oder Naphthyl stehen, oder für einen jeweils gegebenenfalls durch Halogen, Cyano, Nitro, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl substituierten Pyrrol-, Pyrazol-, Imidazol-, Triazol-, Pyridin-, Pyridon-, Pyrimidin-, Pyrazin-, Pyridazin- oder Triazin-Rest stehen, und
- R²: für Wasserstoff, für gegebenenfalls durch Halogen substituiertes C₁-C₆-Alkyl, für gegebenenfalls durch Halogen und/oder C₁-C₄-Alkyl substituiertes C₃-C₆-Cycloalkyl oder für gegebenenfalls durch Halogen oder C₁-C₆-Alkyl substituiertes Phenyl steht.

Nach dem erfindungsgemäßen Verfahren werden insbesondere Verbindungen der Formel (I) hergestellt, in welcher
- R³: für gegebenenfalls durch Halogen, Cyano, Nitro, C₁-C₄-Alkyl, C₁-C₂-Halogenalkyl, C₁-C₄-Alkoxy, C₁-C₂-Halogenalkoxy, C₁-C₄-Alkylthio, C₁-C₂-Halogenalkylthio, C₁-C₄-Alkylsulfinyl, C₁-C₂-Halogenalkylsulfinyl, C₁-C₄-Alkylsulfonyl, C₁-C₂-Halogenalkylsulfonyl, C₁-C₂-Alkylendioxy oder C₁-C₂-Halogenalkylendioxy substituiertes Phenyl steht,
- R²: für Wasserstoff steht und
- R¹: für einen jeweils gegebenenfalls durch Halogen, Cyano, Nitro, C₁-C₄-Alkyl, C₁-C₂-Halogenalkyl, C₁-C₄-Alkoxy, C₁-C₂-Halogenalkoxy, C₁-C₄-Alkylthio oder C₁-C₂-Halogenalkylthio substituierten, über Stickstoff gebundenen Pyrazol- oder Pyridon-Rest steht.

Halogen steht in der Definition der Verbindungen der Formel (I) vorzugsweise für Fluor, Chlor, Brom oder Iod.

Die beim erfindungsgemäßen Verfahren als Zwischenprodukte anfallenden substituierten Dialkylaminoalkylketone der allgemeinen Formel (IV) sind zum Teil aus der Literatur bekannt.

Siehe z.B. Synthetic communications, 17(7), S. 809-815, 1987, worin auch ein Verfahren zur Herstellung dieser Verbindungen durch Reaktion von substituierten Ketonen mit Bisdimethylaminomethan in Gegenwart von Essigsäureanhydrid oder Essigsäure beschrieben wird.

In Formel (IV) haben R¹, R² und R³ vorzugsweise bzw. insbesondere diejenigen Bedeutungen, die bereits oben im Zusammenhang mit der Beschreibung der erfindungsgemäß herzustellenden Verbindungen der Formel (I) vorzugsweise bzw. als insbesondere bevorzugt für R¹, R² und R³ angegeben wurden; R⁴ steht vorzugsweise für C₁-C₆-Alkyl, insbesondere für Methyl, Ethyl, Propyl oder Butyl.

Die beim erfindungsgemäßen Verfahren zur Herstellung von Verbindungen der Formel (I) als Ausgangsstoffe zu verwendenden substituierten Ketone sind durch die Formel (II) allgemein definiert. In Formel (II) haben R¹, R² und R³ vorzugsweise bzw. insbesondere diejenigen Bedeutungen, die bereits oben im Zusammenhang mit der Beschreibung der erfindungsgemäß herzustellenden Verbindungen der Formel (I) vorzugsweise bzw. als insbesondere bevorzugt für R¹, R² und R³ angegeben wurden.

Die Ausgangsstoffe der Formel (II) sind bekannt und/oder können nach an sich bekannten Verfahren hergestellt werden (vgl. EP-A 438690).

Die beim erfindungsgemäßen Verfahren weiter als Ausgangsstoffe einzusetzenden Bis-dialkylaminomethane sind durch die Formel (III) allgemein definiert. In Formel (III) steht R⁴ vorzugsweise für C₁-C₆-Alkyl, insbesondere für Methyl, Ethyl, Propyl oder Butyl.

Die Ausgangsstoffe der Formel (III) sind bekannte organische Synthesechemikalien.

Das erfindungsgemäße Verfahren kann in der ersten Stufe - zur Gewinnung der Zwischenprodukte der Formel (IV) - entweder ohne Zusatz eines Verdünnungsmittels ("in Substanz") oder in Gegenwart eines Verdünnungsmittels durchgeführt werden. Geeignete Verdünnungsmittel sind hierbei insbesondere aliphatische, alicyclische oder aromatische, gegebenenfalls halogenierte Kohlenwasserstoffe, wie z.B. Pentan, Hexan, Heptan, Cyclohexan, Methylcyclohexan, Benzol, Toluol, Xylol, Methylenchlorid, Chloroform, Tetrachlormethan, Chlorbenzol und o-Dichlorbenzol. Die Durchführung mit Methylenchlorid als Verdünnungsmittel wird hierbei ganz besonders bevorzugt.

Die Reaktionstemperaturen können bei der Durchführung der ersten Stufe des erfindungsgemäßen Verfahrens in einem größeren Bereich variiert werden. Man arbeitet bei Temperaturen zwischen 0°C und 100°C, vorzugsweise zwischen 5°C und 80°C, insbesondere zwischen 10°C und 50°C.

Zur Durchführung der ersten Stufe des erfindungsgemäßen Verfahrens setzt man auf 1 Mol substituiertes Keton der Formel (II) im allgemeinen zwischen 0,9 und 1,5 Mol, vorzugsweise zwischen 1,0 und 1,2 Mol, Bis-dialkylaminomethan der Formel (III) ein. Die Ausgangsstoffe der Formeln (II) und (III) - und vorzugsweise ein Verdünnungsmittel wie z.B. Methylenchlorid - werden im allgemeinen bei Temperaturen zwischen ca. 5°C und ca. 30°C vermischt und dann - vorzugsweise bei 30°C bis 100°C - bis zum Ende der Umsetzung gerührt. Die resultierenden Zwischenprodukte der Formel (IV) können im allgemeinen in kristalliner Form isoliert werden, wenn man nach dem Ende der Umsetzung unter vermindertem Druck einengt, den Rückstand mit einem unpolaren Lösungsmittel, wie z.B. Hexan, verrührt und das kristalline Produkt durch Absaugen isoliert. Vorzugsweise wird das Verfahren zur Herstellung der Verbindungen der Formel (I) ohne Zwischenisolierung der Verbindungen (IV) durchgeführt.

Die zweite Stufe des erfindungsgemäßen Verfahrens wird vorzugsweise unter Verwendung eines Verdünnungsmittels durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid, ferner auch Alkohole, wie Methanol, Ethanol, n- und iso-Propanol, n-, iso-, sec- und tert-Butanol, Ethanol wird als Lösungsmittel für die zweite Stufe des erfindungsgemäßen Verfahrens ganz besonders bevorzugt.

Die Reaktionstemperaturen können bei der Durchführung der zweiten Stufe des erfindungsgemäßen Verfahrens in einem größeren Bereich variiert werden. Man arbeitet bei Temperaturen zwischen 0°C und 100°C, vorzugsweise zwischen 0°C und 60°C, insbesondere zwischen 0°C und 40° C.

Zur Durchführung der zweiten Stufe des erfindungsgemäßen Verfahrens setzt man, bezogen auf 1 Mol Ausgangsverbindung der Formel (II), im allgemeinen zwischen 0,9 und 1,5 Mol, vorzugsweise zwischen 1,0 und 1,2 Mol, Hydrazin(hydrat) ein.

In einer ersten Verfahrensvariante wird das wie oben angegebene isolierte Zwischenprodukt der Formel (IV) in einem Verdünnungsmittel, vorzugsweise Ethanol, aufgenommen. Das Hydrazin(hydrat) wird dann langsam zudosiert und das Reaktionsgemisch mehrere Stunden gerührt. Nach Abkühlen kann im allgemeinen das kristallin angefallene Produkt durch Absaugen isoliert werden.

In einer zweiten Verfahrensvariante wird nach Durchführung der ersten Verfahrensstufe das Hydrazin(hydrat) direkt zur Reaktionsmischung gegeben, d.h. auf die Zwischenisolierung des Zwischenprodukt der Formel (IV) wird verzichtet. Die Mischung wird bis zum Ende der Umsetzung gerührt und auf übliche Weise aufgearbeitet (vgl. die Herstellungsbeispiele).

In einer dritten, besonders bevorzugten Verfahrensvariante wird die erste Verfahrensstufe wie oben angegeben mit Methylenchlorid als Verdünnungsmittel durchgeführt. Dann wird eingeengt, der Rückstand in Ethanol aufgenommen und das Hydrazin(hydrat) dazugegeben. Das Reaktionsgemisch wird dann bis zum Ende der Umsetzung gerührt. Nach Abkühlen kann das Produkt durch Absaugen isoliert werden. Es kann aber auch vorteilhaft sein, nach dem Ende der Umsetzung einzuengen, den Rückstand mit einem anderen Lösungsmittel, wie z.B. Wasser, zu verrühren und abzusaugen (vgl. die Herstellungsbeispiele).

Die nach dem erfindungsgemäßen Verfahren herzustellenden substituierten Pyrazoline der Formel (I) können als Zwischenprodukte zur Herstellung von Schädlingsbekämpfungsmitteln verwendet werden (vgl. EP-A 438690).

Die Umsetzung einer Verbindung der Formel (I) zum insektiziden Endprodukt sei am folgendem Beispiel erläutert:

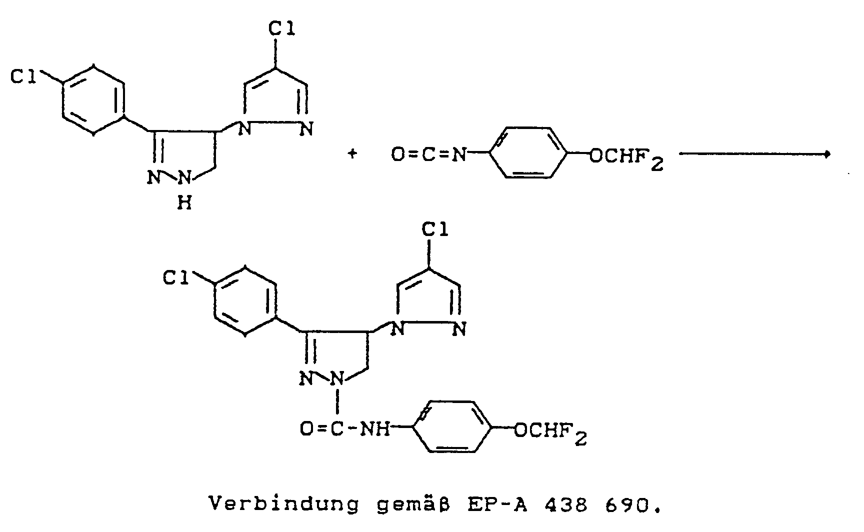

### Herstellungsbeispiele:

### Beispiel 1

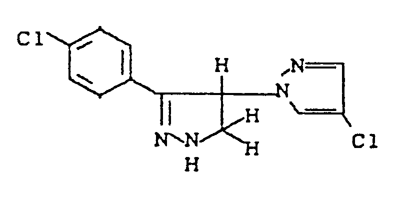

107 g (1,05 Mol) Bis-dimethylaminomethan werden zu einer Lösung von 255 g (1,0 Mol) α-(4-Chlor-pyrazol-1-yl)-4-chlor-acetophenon in 900 ml Dichlormethan bei 20°C bis 25°C gegeben. Dann wird das Gemisch 90 Minuten unter Rückfluß erhitzt. Nach Abkühlen auf 20°C werden 55 g (1,1 Mol) Hydrazinhydrat innerhalb von 15 Minuten zugetropft. Das Reaktionsgemisch wird dann 20 Stunden bei 20°C und schließlich 30 Minuten bei 5°C gerührt. Das kristallin angefallene Produkt wird durch Absaugen isoliert.

Man erhält 115 g (41% der Theorie) 3-(4-Chlor-phenyl)-4-(4-chlor-pyrazol-1-yl)-4,5-dihydro-1H-pyrazol vom Schmelzpunkt 176°C.

Das Filtrat wird durchgeschüttelt, die organische Phase abgetrennt, zweimal mit je 200 ml Wasser gewaschen, mit Natriumsulfat getrocknet und filtriert. Das Filtrat wird im Wasserstrahlvakuum eingeengt und der Rückstand mit Diisopropylether verrührt. Das hierbei kristallin angefallene Produkt wird durch Absaugen isoliert.

Man erhält so weitere 47,2 g (17% der Theorie) des oben genannten Produktes. Gesamt-Ausbeute: 162,2 g (58% der Theorie).

### Beispiel 2

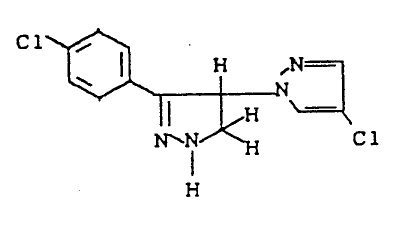

450 g (4,4 Mol) Bis-dimethylaminomethan werden bei 20°C bis 25°C zu einer Mischung aus 1020 g (4,0 Mol) α-(4-Chlor-pyrazol-1-yl)-4-chlor-acetophenon und 1600 ml Methylenchlorid gegeben. Das Reaktionsgemisch wird 15 Stunden unter Rückfluß erhitzt und anschließend im Wasserstrahlvakuum eingeengt. Der verbleibende Rückstand wird in 4 Liter Ethanol aufgenommen; dann werden 232 g (4,64 Mol) Hydrazinhydrat innerhalb von 15 Minuten tropfenweise dazugegeben. Nach ca. 30 Minuten bildet sich eine klare Lösung; etwa nach weiteren 30 Minuten beginnt die Kristallisation des Produktes. Die Mischung wird ca. 5 Stunden bei 25°C bis 30°C und dann ca. 30 Minuten bei 10°C gerührt; dann wird das kristalline Produkt durch Absaugen isoliert.

Man erhält 983 g (87% der Theorie) 3-(4-Chlor-phenyl)-4-(4-chlor-pyrazol-1-yl)-4,5-dihydro-1H-pyrazol vom Schmelzpunkt 176°C.

### Beispiel 3

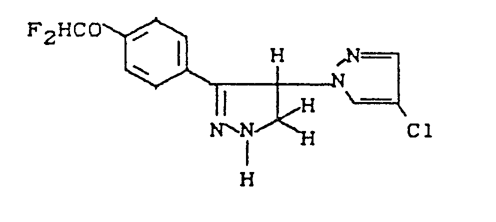

56,2 g (0,55 Mol) Bis-dimethylaminomethan werden bei 20°C zu einer Mischung aus 143 g (0,5 Mol) α-(4-Chlorpyrazol-1-yl)-4-difluormethoxy-acetophenon und 450 ml Methylenchlorid gegeben. Das Reaktionsgemisch wird 19 Stunden unter Rückfluß erhitzt und anschließend im Wasserstrahlvakuum eingeengt. Der verbleibende Rückstand wird in 750 ml Ethanol aufgenommen und mit 27 g (0,54 Mol) Hydrazinhydrat versetzt, woraufhin die Innentemperatur von 25°C auf 35°C ansteigt. Das Reaktionsgemisch wird noch 5 Stunden gerührt und anschließend im Wasserstrahlvakuum eingeengt. Der Rückstand wird mit 1 Liter Wasser verrührt und das kristalline Produkt durch Absaugen isoliert.

Man erhält 154 g (98% der Theorie) 3-(4-Difluormethoxyphenyl)-4-(4-chlor-pyrazol-1-yl)-4,5-dihydro-1H-pyrazol vom Schmelzpunkt 93°C.

### Zwischenprodukte der Formel (IV)

### Beispiel (IV-1)

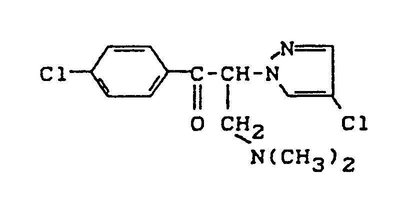

Eine Mischung aus 143 g (0,56 Mol) α-(4-Chlor-pyrazol-1-yl)-4-chlor-acetophenon, 63 g (0,62 Mol) Bis-dimethylaminomethan und 500 ml Methylenchlorid wird 15 Stunden unter Rückfluß erhitzt und anschließend im Wasserstrahlvakuum eingeengt. Der Rückstand wird mit 500 ml Hexan verrührt und das kristallin angefallene Produkt durch Absaugen isoliert.

Man erhält 148 g (85% der Theorie) α-(4-Chlor-pyrazol-1-yl)-α-dimethylaminomethyl-4-chlor-acetophenon vom Schmelzpunkt 107°C.

## Patentansprüche

1. Verfahren zur Herstellung von substituierten Pyrazolinen der allgemeinen Formel (I) 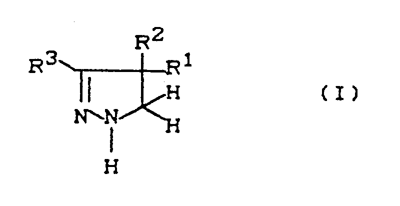 in welcher
R¹ und R³ gleich oder verschieden sind und jeweils für gegebenenfalls substituiertes Aryl oder für gegebenenfalls substituiertes und/oder benzanelliertes Heterocyclyl mit 5 oder 6 Ringgliedern, von denen 1 bis 4 Stickstoffatome und die restlichen Kohlenstoffatome sind, stehen, und
R² für Wasserstoff oder für jeweils gegebenenfalls substituiertes Alkyl, Cycloalkyl oder Aryl steht,
dadurch gekennzeichnet, daß man in einer ersten Stufe eine Mischung bestehend aus einem substituierten Keton der allgemeinen Formel (II) 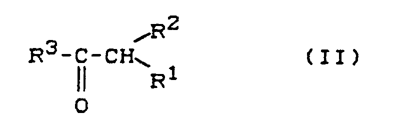 in welcher
R¹, R² und R³ die oben angegebene Bedeutung haben,
und einem Bis-dialkylaminomethan der allgemeinen Formel (III)
(R⁴)₂N-CH₂-N(R⁴)₂ (III)
in welcher
R⁴ für Alkyl steht,
gegebenenfalls in Gegenwart eines Verdünnungsmittels bei Temperaturen zwischen 0°C und 100°C umsetzt und die hierbei gebildeten substituierten Dialkylaminoalkylketone der allgemeinen Formel (IV) 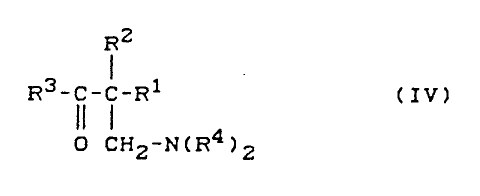 in welcher
R¹, R², R³ und R⁴ die oben angegebene Bedeutung haben,
gegebenenfalls isoliert und in einer zweiten Verfahrensstufe mit Hydrazin(hydrat) gegebenenfalls in Gegenwart eines Verdünnungsmittels bei Temperaturen zwischen 0°C und 100°C umsetzt.

2. Verfahren gemäß Anspruch 1, dadurch gekennzeichnet, daß das Dialkylaminoalkylketon-Zwischenprodukt der Formel (IV) nicht isoliert wird.

3. Verfahren zur Herstellung von substituierten Pyrazolinen der allgemeinen Formel (I) gemäß Anspruch 1) und 2), in welcher
R¹ und R³ gleich oder verschieden sind und jeweils für gegebenenfalls durch Halogen, Cyano, Nitro, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Di-(C₁-C₄-alkyl)-amino, C₁-C₆-Alkoxy-carbonyl, C₁-C₆-Halogenalkoxy-carbonyl, C₁-C₂-Alkylendioxy, C₁-C₂-Halogenalkylendioxy, Phenoxy (welches gegebenenfalls durch Halogen und/oder C₁-C₄-Halogenalkyl substituiert ist) oder Phenylthio (welches gegebenenfalls durch Halogen und/oder C₁-C₄-Halogenalkyl substituiert ist) substituiertes Phenyl oder Naphthyl stehen, oder für einen jeweils gegebenenfalls durch Halogen, Cyano, Nitro, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl substituierten Pyrrol-, Pyrazol-, Imidazol-, Triazol-, Pyridin-, Pyridon-, Pyrimidin-, Pyrazin-, Pyridazin-oder Triazin-Rest stehen, und
R² für Wasserstoff, für gegebenenfalls durch Halogen substituiertes C₁-C₆-Alkyl, für gegebenenfalls durch Halogen und/oder C₁-C₄-Alkyl substituiertes C₃-C₆-Cycloalkyl oder für gegebenenfalls durch Halogen oder C₁-C₆-Alkyl substituiertes Phenyl steht.

4. Verfahren zur Herstellung von substituierten Pyrazolinen der allgemeinen Formel (I) gemäß Anspruch 1) und 2), in welcher
R³ für gegebenenfalls durch Halogen, Cyano, Nitro, C₁-C₄-Alkyl, C₁-C₂-Halogenalkyl, C₁-C₄-Alkoxy, C₁-C₂-Halogenalkoxy, C₁-C₄-Alkylthio, C₁-C₂-Halogenalkylthio, C₁-C₄-Alkylsulfinyl, C₁-C₂-Halogenalkylsulfinyl, C₁-C₄-Alkylsulfonyl, C₁-C₂-Halogenalkylsulfonyl, C₁-C₂-Alkylendioxy oder C₁-C₂-Halogenalkylendioxy substituiertes Phenyl steht,
R² für Wasserstoff steht und
R¹ für einen jeweils gegebenenfalls durch Halogen, Cyano, Nitro, C₁-C₄-Alkyl, C₁-C₂-Halogenalkyl, C₁-C₄-Alkoxy, C₁-C₂-Halogenalkoxy, C₁-C₄-Alkylthio oder C₁-C₂-Halogenalkylthio substituierten, über Stickstoff gebundenen Pyrazol- oder Pyridon-Rest steht.

5. Verfahren gemäß Anspruch 1) bis 4), dadurch gekennzeichnet, daß die erste Reaktionsstufe zwischen 5 und 80°C durchgeführt wird.

6. Verfahren gemäß Anspruch 1) bis 5), dadurch gekennzeichnet, daß man pro Mol substituiertes Keton der Formel (II) zwischen 0,9 und 1,5 Mol Bis-dialkylaminomethan der Formel (III) einsetzt.

7. Verfahren gemäß Anspruch 1) bis 6), dadurch gekennzeichnet, daß man die zweite Stufe in Gegenwart eines inerten organischen Verdünnungsmittels bei Temperaturen zwischen 0°C und 60°C durchführt.

8. Verfahren gemäß Anspruch 1) bis 7), dadurch gekennzeichnet, daß man pro Mol Ausgangsverbindung der Formel (II) zwischen 0,9 und 1,5 Mol Hydrazin(hydrat) einsetzt.

9. Substituiertes Dialkylaminoalkylketon der Formel

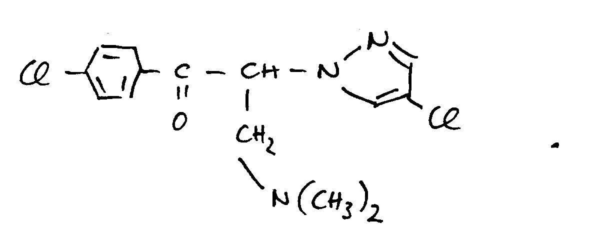

## Claims

1. Process for the preparation of substituted pyrazolines of the general formula (I) 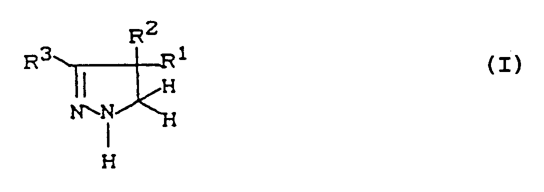 in which
R¹ and R³ are identical or different and each represent optionally substituted aryl or optionally substituted and/or benzo-fused heterocyclyl with 5 or 6 ring members of which 1 to 4 are nitrogen atoms and the remainder are carbon atoms, and
R² represents hydrogen or alkyl, cycloalkyl or aryl, each of which is optionally substituted,
characterised in that, in a first stage, a mixture consisting of a substituted ketone of the general formula (II) 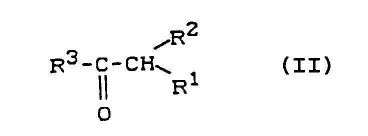 in which
R¹, R² and R³ have the abovementioned meaning,
and a bis-dialkylaminomethane of the general formula (III)
(R⁴)₂N-CH₂-N(R⁴)₂ (III)
in which
R⁴ represents alkyl,
is reacted,
optionally in the presence of a diluent, at temperatures between 0°C and 100°C and the substituted dialkylaminoalkyl ketones of the general formula (IV) 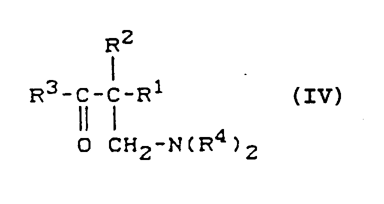 in which
R¹, R², R³ and R⁴ have the abovementioned meaning,
formed in this way are optionally isolated and, in a second process stage, are reacted with hydrazine (hydrate), optionally in the presence of a diluent, at temperatures between 0°C and 100°C.

2. Process according to Claim 1, characterised in that the dialkylaminoalkyl ketone intermediate of the formula (IV) is not isolated.

3. Process for the preparation of substituted pyrazolines of the general formula (I) according to Claims 1) and 2), in which
R¹ and R³ are identical or different and each represent phenyl or naphthyl, each of which is optionally substituted by halogen, cyano, nitro, C₁-C₆-alkyl, C₁-C₆-halogenoalkyl, C₁-C₆-alkoxy, C₁-C₆-halogenoalkoxy, C₁-C₆-alkylthio, C₁-C₆-halogenoalkylthio, C₁-C₆-alkylsulphinyl, C₁-C₆-halogenoalkylsulphinyl, C₁-C₆-alkylsulphonyl, C₁-C₆-halogenoalkylsulphonyl, di-(C₁-C₄-alkyl)-amino, C₁-C₆-alkoxycarbonyl, C₁-C₆-halogenoalkoxycarbonyl, C₁-C₂-alkylenedioxy, C₁-C₂-halogenoalkylenedioxy, phenoxy (which is optionally substituted by halogen and/or C₁-C₄-halogenoalkyl) or phenylthio (which is optionally substituted by halogen and/or C₁-C₄-halogenoalkyl), or each represent a pyrrole, pyrazole, imidazole, triazole, pyridine, pyridone, pyrimidine, pyrazine, pyridazine or triazine radical, each of which is optionally substituted by halogen, cyano, nitro, C₁-C₆-alkyl, C₁-C₆-halogenoalkyl, C₁-C₆-alkoxy, C₁-C₆-halogenoalkoxy, C₁-C₆-alkylthio, C₁-C₆-halogenoalkylthio, C₁-C₆-alkylsulphinyl, C₁-C₆-halogenoalkylsulphinyl, C₁-C₆-alkylsulphonyl or C₁-C₆-halogenoalkylsulphonyl, and
R² represents hydrogen, C₁-C₆-alkyl optionally substituted by halogen, C₃-C₆-cycloalkyl optionally substituted by halogen and/or C₁-C₄-alkyl or phenyl optionally substituted by halogen or C₁-C₆-alkyl.

4. Process for the preparation of substituted pyrazolines of the general formula (I) according to Claims 1) and 2), in which
R³ represents phenyl, optionally substituted by halogen, cyano, nitro, C₁-C₄-alkyl, C₁-C₂-halogenoalkyl, C₁-C₄-alkoxy, C₁-C₂-halogenoalkoxy, C₁-C₄-alkylthio, C₁-C₂-halogenoalkylthio, C₁-C₄-alkylsulphinyl, C₁-C₂-halogenoalkylsulphinyl, C₁-C₄-alkylsulphonyl, C₁-C₂-halogenoalkylsulphonyl, C₁-C₂-alkylenedioxy or C₁-C₂-halogenoalkylenedioxy,
R² represents hydrogen, and
R¹ represents a pyrazole or pyridone radical, each of which is bonded via nitrogen and is optionally substituted by halogen, cyano, nitro, C₁-C₄-alkyl, C₁-C₂-halogenoalkyl, C₁-C₄-alkoxy, C₁-C₂-halogenoalkoxy, C₁-C₄-alkylthio or C₁-C₂-halogenoalkylthio.

5. Process according to Claims 1) to 4), characterised in that the first reaction stage is performed between 5°C and 80°C.

6. Process according to Claims 1) to 5), characterised in that between 0.9 and 1.5 mol of bis-dialkylaminomethane of the formula (III) is used per mole of substituted ketone of the formula (II).

7. Process according to Claims 1) to 6), characterised in that the second stage is performed in the presence of an inert organic diluent at temperatures between 0°C and 60°C.

8. Process according to Claims 1) to 7), characterised in that between 0.9 and 1.5 mol of hydrazine (hydrate) is used per mole of starting compound of the formula (II).

9. Substituted dialkylaminoalkyl ketone of the formula

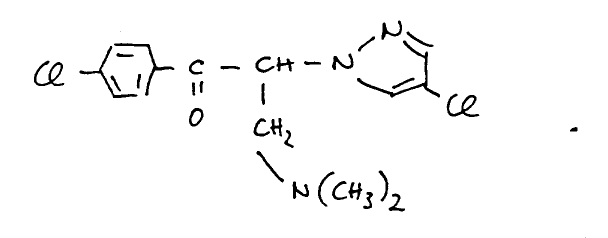

## Revendications

1. Procédé pour la préparation de pyrazolines substituées répondant à la formule générale (I) 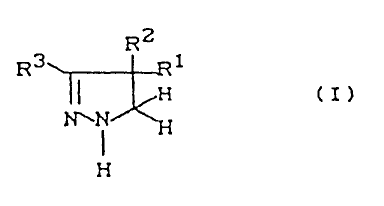 dans laquelle
R¹ et R³ sont identiques ou différents et représentent respectivement un groupe aryle éventuellement substitué ou un groupe hétérocyclyle éventuellement substitué et/ou benzocondensé contenant 5 ou 6 termes cycliques dont 1 à 4 termes représentent des atomes d'azote et les termes restants représentent des atomes de carbone, et
R² représente un atome d'hydrogène ou encore un groupe alkyle, un groupe cycloalkyle ou un groupe aryle, chacun de ces groupes portant éventuellement un ou plusieurs substituants identiques ou différents,
caractérisé en ce que, dans une première étape, on fait réagir un mélange constitué par une cétone substituée répondant à la formule générale (II) 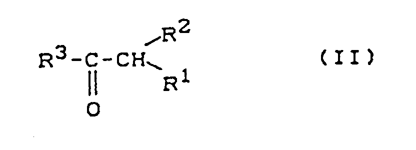 dans laquelle
R¹, R² et R³ ont la signification indiquée ci-dessus,
et par un bisdialkylaminométhane répondant à la formule générale (III)
(R⁴)₂N-CH₂-N(R⁴)₂ (III)
dans laquelle
R⁴ représente un groupe alkyle,
éventuellement en présence d'un diluant, à des températures entre 0°C et 100°C, et on isole éventuellement les dialkylaminoalkylcétones substituées que l'on obtient en l'occurrence, répondant à la formule générale (IV) 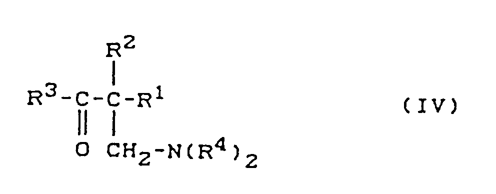 dans laquelle
R¹, R², R³ et R⁴ ont la signification indiquée ci-dessus,
et dans une seconde étape opératoire, on les fait réagir avec de l' (hydrate d')hydrazine, éventuellement en présence d'un diluant, à des températures entre 0°C et 100°C.

2. Procédé selon la revendication 1, caractérisé en ce qu'on n'isole pas le produit intermédiaire de dialkylaminoalkylcétone de formule (IV).

3. Procédé pour la préparation de pyrazolines substituées répondant à la formule générale (I) selon les revendications 1) et 2), dans laquelle
R¹ et R³ sont identiques ou différents et représentent respectivement un groupe phényle ou un groupe naphtyle portant éventuellement un ou plusieurs substituants identiques ou différents halogéno, cyano, nitro, alkyle en C₁-C₆, halogénalkyle en C₁-C₆, alcoxy en C₁-C₆, halogénalcoxy en C₁-C₆, alkyl(en C₁-C₆)thio, halogénalkyl(en C₁-C₆)thio, alkyl(en C₁-C₆)sulfinyle, halogénalkyl(en C₁-C₆)sulfinyle, alkyl(en C₁-C₆)sulfonyle, halogénalkyl(en C₁-C₆)sulfonyle, di(alkyl en C₁-C₄)amino, alcoxy(en C₁-C₆)carbonyle, halogénalcoxy(en C₁-C₆)carbonyle, alkylène(en C₁-C₂)dioxy, halogénalkylène(en C₁-C₂)dioxy, phénoxy (qui porte éventuellement un ou plusieurs substituants identiques ou différents halogéno et/ou halogénalkyle en C₁-C₄) ou phénylthio (qui porte éventuellement un ou plusieurs substituants identiques ou différents halogéno et/ou halogénalkyle en C₁-C₄); ou encore un radical pyrrole, un radical pyrazole, un radical imidazole, un radical triazole, un radical pyridine, un radical pyridone, un radical pyrimidine, un radical pyrazine, un radical pyridazine ou un radical triazine, chacun de ces radicaux portant éventuellement un ou plusieurs substituants identiques ou différents halogéno, cyano, nitro, alkyle en C₁-C₆, halogénalkyle en C₁-C₆, alcoxy en C₁-C₆, halogénalcoxy en C₁-C₆, alkyl(en C₁-C₆)thio, halogénalkyl(en C₁-C₆)thio, alkyl(en C₁-C₆)sulfinyle, halogénalkyl(en C₁-C₆)sulfinyle, alkyl(en C₁-C₆)sulfonyle ou halogénalkyl(en C₁-C₆)sulfonyle, et
R² représente un atome d'hydrogène; un groupe alkyle en C₁-C₆ portant éventuellement un ou plusieurs substituants halogéno identiques ou différents; un groupe cycloalkyle en C₃-C₆ portant éventuellement un ou plusieurs substituants identiques ou différents halogéno et/ou alkyle en C₁-C₄; ou encore un groupe phényle portant éventuellement un ou plusieurs substituants identiques ou différents halogéno ou alkyle en C₁-C₆.

4. Procédé pour la préparation de pyrazolines substituées répondant à la formule générale (I) selon les revendications 1) et 2), dans laquelle
R³ représente un groupe phényle portant éventuellement un ou plusieurs substituants identiques ou différents halogéno, cyano, nitro, alkyle en C₁-C₄, halogénalkyle en C₁-C₂, alcoxy en C₁-C₄, halogénalcoxy en C₁-C₂, alkyl(en C₁-C₄)thio, halogénalkyl(en C₁-C₂)thio, alkyl(en C₁-C₄)sulfinyle, halogénalkyl(en C₁-C₂)sulfinyle, alkyl(en C₁-C₄)sulfonyle, halogénalkyl(en C₁-C₂)sulfonyle, alkylène(en C₁-C₂)dioxy ou halogénalkylène(en C₁-C₂)dioxy,
R² représente un atome d'hydrogène et
R¹ représente un radical pyrazole ou un radical pyridone lié par un atome d'azote portant respectivement éventuellement un ou plusieurs substituants identiques ou différents halogéno, cyano, nitro, alkyle en C₁-C₄, halogénalkyle en C₁-C₂, alcoxy en C₁-C₄, halogénalcoxy en C₁-C₂, alkyl(en C₁-C₄)thio ou halogénalkyl(en C₁-C₂)thio.

5. Procédé selon les revendications 1 à 4, caractérisé en ce qu'on effectue la première étape réactionnelle entre 5 et 80°C.

6. Procédé selon les revendications 1 à 5, caractérisé en ce que, par mole de cétone substituée de formule (II), on met en oeuvre entre 0,9 et 1,5 mole de bisdialkylaminométhane de formule (III).

7. Procédé selon les revendications 1 à 6, caractérisé en ce qu'on effectue la seconde étape en présence d'un diluant organique inerte à des températures entre 0°C et 60°C.

8. Procédé selon les revendications 1 à 7, caractérisé en ce que, par mole de composé de départ de formule (II), on met en oeuvre entre 0,9 et 1,5 mole d'(hydrate d')hydrazine.

9. Dialkylaminoalkylcétone substituée de formule
